# EUROPEAN PATENT SPECIFICATION

(11) **EP 1 993 433 B1**
(45) Date of publication and mention of the grant of the patent: **01.09.2010**
(21) Application number: 07705943.4
(22) Date of filing: 26.02.2007
(51) Int. Cl.: A61B 5/00

(54) **BODY PARAMETER SENSING**
KÖRPERPARAMETERERFASSUNG
DETECTION DE PARAMETRES CORPORELS

(30) Priority: 02.03.2006 EP 06110583
(43) Date of publication of application: 26.11.2008
(73) Proprietor: Koninklijke Philips Electronics N.V., 5621 BA Eindhoven (NL)
(72) Inventor: COENE, Willem, M., J., M., NL-5656 AA Eindhoven (NL); OUWERKERK, Martin, NL-5656 AA Eindhoven (NL)
(74) Representative: Schouten, Marcus Maria
(86) International application number: PCT/IB2007/050598
(87) International publication number: WO 2007/099487

(56) References cited:
- WO-A-2005/089642
- US-A1- 2002 120 202

## Description

The invention relates to a body parameter sensing arrangement and a method of sensing body parameters.

It has been suggested to incorporate sensors for such things as ECG (Electrocardiogram) signals in clothing, without fixed attachment to the body. The person whose body signals are measured merely needs to don the piece of clothing to enable sensing. This makes it possible to monitor body signals of the person during normal activities (i.e. activities that are not specifically directed at measuring body signals), without encumbering the person by the attachment of sensors to the body.

In such an arrangement of sensors that are attached to flexible clothing, the sensors are able to move relative to each other. Unfortunately, it has been found that such an arrangement with sensors in the clothing may have the effect that some sensors will temporarily fail to provide relevant signals, for example when a body movement temporarily creates a distance between the sensor and the body. Therefore it is desirable to provide for a way of eliminating those sensors that do not provide valid signals at a given moment in time and to adapt the set of eliminated sensors as a function of time.

US patent application No 2002/0120202 describes a heartbeat sensor. This sensor comprises a linear array of pressure sensors pushed against the wrist. The pressure sensors are located at fixed positions with respect to each other in the array, all in permanent contact with the wrist (at least when the heartbeat sensor is worn), so that some are pressed against the artery and other press against the wrist adjacent the artery. The fixed array of sensors is used to provide signals with and without prominent heartbeat pressure. The latter are subtracted from the signals with prominent heartbeat pressure. This eliminates the effect of pressure changes due to flexing of muscles, body movement such as running etc.

US patent application No 2002/0120202 provides for a dynamic selection of the sensors that provide heartbeat signals. Selection involves cross correlating of signals from adjacent sensors. Details are not given, but the document mentions in its background art section that US patent No 5,243,993 used a correlation function to accept a heartbeat signal.

US patent No 5,243,993 describes the computation of a correlation coefficient between the signals for a current (surmised) heartbeat and a previous heartbeat. The surmised heartbeat is accepted as a real heartbeat if the correlation coefficient is within a predetermined range.

It should be noted that these documents assume the use of a linear array of pressure sensors, located at mechanically fixed positions relative to one another. This arrangement defines the signal processing problem: it ensures that, going along the array, successively first some sensors will not sense direct pressure from the artery, then some sensors will sense direct pressure and finally some sensors not sense direct pressure, and that signals from all sensors will have a similar common mode background signal. Furthermore it ensures that the heartbeat is present simultaneously in all signals in which it is present. The processing of the signals is designed to make use of these relations between the signals from the different sensors in the array.

US patent No 5,243,993 and US patent application No 2002/0120202 are not concerned with sensor configurations in clothing that allows independent relative movement between the sensors in the configuration. These documents are also not concerned with capacitive sensors of electric body potentials.

Among others, it is an object to provide for a body parameter sensing arrangement with a plurality of sensors that are able to move relative to one another and the body and in which signals from sensors that temporarily provide insufficient information can be eliminated.

The invention provides for a body parameter sensing arrangement according to claim 1. This arrangement comprises clothing and a plurality of sensors for sensing body signals, located at mutually movable relative positions in the clothing. The sensors may be capacitive electrodes for example, for capacitively picking up body potentials corresponding to electrocardiogram data (ECG).

A processing circuit identifies selected ones of the sensors that carry valid body signals by clustering the sensors according to a measure of similarity between signals from the sensors. Similarity is measured for example by means of decreasing difference between sensor signals or correlation between the signals. Sensors with fading signals will have a significantly noisy-type character, which does not correlate to the body signal of interest, leading to less similarity with signals from sensors that do carry valid body signals.

A cluster of sensors is selected with a maximal number of sensors with a minimal cluster diameter. The cluster diameter is defined by a maximum value of a distance measure between signals for any pair of sensors in the cluster, or of any decreasing function of a minimum value of correlations between signals for any pair of sensors in the cluster for example. One or more sensors are selected as sensors carrying valid body signals on the basis of their membership of the selected cluster. In an example, a cluster of with a maximum count of sensors and a diameter less than a threshold is used to select sensors that carry valid body signals. In this way sensors that do not receive reliable signals from the body are eliminated.

In an embodiment the similarity or distance is computed from signals with mutual time offset that accounts for differences in travel time to the locations on the body where the different sensors pick up signals. In one embodiment predetermined time offsets may be used, that account for the different locations. In another embodiment the time offsets are adapted, so that changes in sensing location due to movement of the clothing can be accounted for.

These and other objects and advantageous aspects will become apparent from a description of exemplary embodiments, using the following figures.
Figure 1 shows an example of sensor locations in a piece of clothing;
Figure 2 shows a circuit comprising sensors in clothing;
Figure 3 shows a flow-chart of processing of signals from sensors.

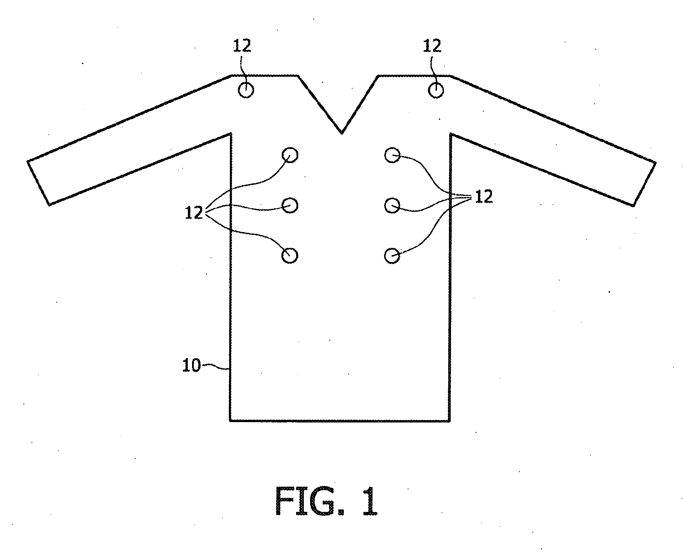

Figure 1 shows a piece of clothing 10 with sensor locations 12 of capacitive sensors. In one example the sensors comprise capacitive electrode plates glued or stitched to the piece of clothing, or incorporated in pockets in the piece of clothing.

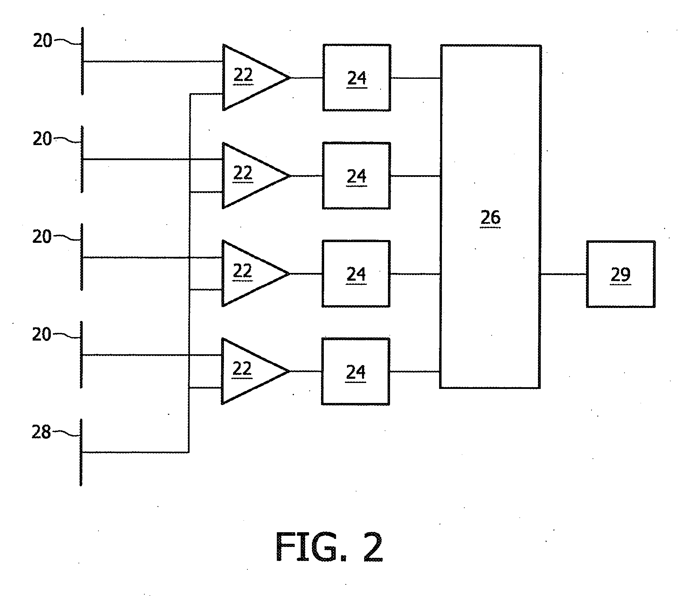

Figure 2 shows a circuit comprising electrode plates 20 from the piece of clothing. The circuit comprises differential amplifiers 22 for respective ones of the electrode plates, A/D (Analog to Digital) conversion circuits 24, a digital signal processing circuit 26 and a memory 29. Each differential amplifier 22 has inputs coupled to a reference electrode plate 28 and a respective one of the electrode plates 20 and an output coupled to an input of a respective one of the A/D conversion circuits 24. Each A/D conversion circuit has an output coupled to digital signal processing circuit 26. Memory 29 is coupled to digital signal processing circuit 26.

It should be appreciated that the circuit is merely one representative circuit for a sensor arrangement. Many variations are possible, for example, instead of using separate inputs to digital signal processing circuit 26 some small network may be used to interconnect digital signal processing circuit 26 and A/D converters 24. Similarly, the connection of amplifiers 22 to reference electrode 28 may be implicit when reference electrode 28 is coupled to circuit ground. Also, instead of a single reference electrode 28, different reference electrodes may be coupled to the reference inputs of respective ones of amplifiers 22.

In one example operation, the circuit functions to record electrocardiograms of the person that wears the piece of clothing in memory 29. As is well known, heart activity causes time variable electric potentials on the body surface from which an electrocardiogram can be obtained. Conventionally, this is done by fixedly attaching electrodes to the body of a patient and recording signals from the electrodes. As an alternative it is proposed to use electrodes in the piece of clothing, without fixed attachment to the body. In this example, at any one time only a single signal from one of the capacitive electrodes suffices, but it does not suffice to use one and the same capacitive electrode all the time, because movement of the person that wears the piece of clothing can temporarily remove a capacitive electrode from being in close contact with the body. Therefore, a method is needed for dynamical selection from the capacitive electrodes to identify electrodes that provide representative signals at each relevant time point.

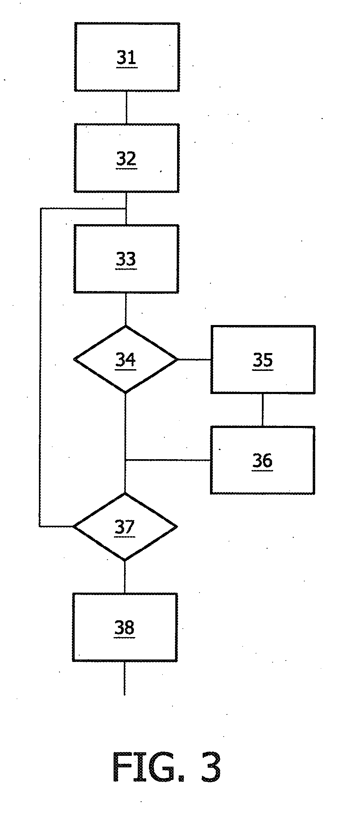

Figure 3 shows a flow-chart of operations of signal processing circuit 26 to select signals from electrode plates 20. In a first step 31 signal processing circuit 26 receives signals R(i,t) for time points t a time interval from A/D converters 24 (labeled by i=1,2,3 etc.). A set of time-sequences R(i,t) of time samples t during a predetermined time-interval is the input of the algorithm. The time interval may have a duration of a heartbeat period for example, but it may also have a duration that is several heartbeat periods long, or even longer, or shorter when other signals than heartbeats need to be monitored. In another embodiment the time interval is variable, adaptive to the heartbeat period. In a second step 32 signal processing circuit 26 computes correlation coefficients C(i,j) between the signals of A/D converters 24 for different electrodes, corresponding to a sum over time of products R(i,t)xR(j,t') of signals for different sensors labeled by i and j (x standing for multiplication of the values R(i,t) and R(j,t')). Herein t'=t+dt wherein dt is an offset value for which the correlation is computed. In one embodiment dt equals zero, but as will be described other values may be used. The sum over time values is performed over a time-span determined by the length of the earlier mentioned time-interval. Said sum is normalized by the square roots of similar sums for auto correlations R(i,t)x R(i,t) and R(j,t)x R(j,t).

In a third step 33 signal processing circuit 26 starts eliminating signals from a cluster of accepted electrodes. In initial execution third step 33 signal processing circuit 26 starts with a cluster of accepted electrodes, which initially contains all electrodes. In successive executions processing circuit 26 selects successive pairs of electrodes i, j.

In a fourth step 34 signal processing circuit 26 tests whether the correlation coefficients between the signals from the pair of electrodes is below a predetermined threshold. If so, signal processing circuit 26 executes a fifth step 35, determining counts for electrodes i and j of the number of other electrodes in the cluster of accepted electrodes that have correlations above a further threshold with electrode i and j respectively. Subsequently signal processing circuit 26 executes a sixth step 36, removing the electrode i or j from the pair with the lowest count from the cluster of accepted electrodes. Then signal processing circuit 26 executes a seventh step 37, testing whether all pairs in the cluster of accepted cluster have been visited. If not, signal processing circuit 26 returns to third step 33, selecting another pair from the cluster of accepted electrodes. When, in fourth step 34 signal processing circuit 26 determines that the correlation is sufficiently high, signal-processing circuit 26 proceeds to seventh step 37 directly.

When signal processing circuit 26 determines in seventh step 37 that all pairs have been visited, signal processing circuit 26 proceeds to eight step 38, selecting one of the accepted signals for storage of a result. In an alternative embodiment the signals from the cluster of accepted signals are averaged for time values in the time interval under consideration and the resulting averages are stored. The stored signals may be held available for later diagnostic inspection by a doctor, or for use by an analysis computer to perform a computation detect unusual signal shapes for example.

After eight step 38 signal processing circuit 26 repeats from first step 31 for another time interval. In an embodiment, this is a time interval that immediately follows the time interval for which the steps of the flow-chart were performed previously. In another embodiment consecutively overlapping time intervals may be used. In an alternative embodiment eight step 38 is performed for a plurality of time intervals using the same cluster of accepted signals, until any correlation between the signals in the cluster drops below a threshold value, or until a predetermined number of time intervals has been processed. This reduces the required amount of processing. This is possible because the cluster of accepted signals typically will change only at a much slower rate than after each time interval.

Preferably, in second step 32 the correlation coefficients C(i,j) are computed by summing products R(i,t)xR(j,t') over time values t; t' selected so that t-t' is a fixed offset value dt for the pair of electrodes i, j, which corresponds to the difference in traveling time of the ECG signals to the respective points on the body from which the electrodes i and j pick up signals.

In one embodiment (a program of) digital processing circuit 26 defines predetermined values of the offset values for respective ones if the pairs of electrodes i, j and uses these offset values dt(i,j) in the computation of the correlations, where the offset values dt(i,j) for a pair i, j may differ from zero. The predetermined offset values dt(i,j) corresponds to differences between traveling time to the locations 12 of the electrodes of the pair on the piece of clothing. This embodiment is based on the assumption that movement of the piece of clothing, with attendant changes in location of the electrodes, does not allow significant changes in the traveling time through the body to the location of the electrodes.

In an alternative embodiment dynamically determined values of the offset values dt(i,j) for different pairs of electrodes are used. This may be realized for example by computing the correlation coefficient for each pair for a range of offset values dt around a nominal value dt0 for the pair and selection of the correlation for the offset value that yields a maximum value of the correlation. This type of cross-correlations can be very effectively computed via Fast-Fourier transforms. Herein the nominal value dt0(i,j) for the pair i, j corresponds to differences between nominal traveling time distances to the locations 12 of the pair of electrodes. In another embodiment dynamically determined values of the offset values dt(ij) are selected for a plurality of successive executions of the flow-chart, by periodic searching of offset values dt(i,j) that lead to maximum correlation and subsequent repeated use of these offset values dt(i,j).

In an embodiment the predetermined or dynamically selected offset values dt(i,j) are also used for averaging the accepted signals, for example by averaging the signals each delayed by its offset value dt with respect to a reference one of the signals.

In a further embodiment the signals R(i,t+dt) may be filtered with a filter having a response function f(i,t) of time, said filtering operation being carried out before averaging.

It should be appreciated that the described method of eliminating signals from the cluster of accepted signals is only one example of elimination of unreliable signals.

The algorithm is an approximate solution to the problem of finding a cluster with a diameter below the threshold value that has the highest cardinality. Herein the "cardinality" of the cluster is the number of sensors in the cluster. The "diameter" to the maximum distance between the signals from any pair of sensors in the cluster. The correlation between signals is indicative of the distance and therefore for the diameter: distance increases with decreasing correlation.

It will be appreciated that other types of distance measure may be used for defming diameter, leading to different embodiments. Distance measures between signals are known per se. A sum over time t of squares of differences R(i,t)-R(j,t') may be used for example. Instead of a sum of squares of signal values for different time points for example a sum of absolute values of differences may be used, or of powers of such absolute values, or a weighted sum, or sums of spectral differences etc. Also instead of a largest cluster in an algorithm independent sense, a largest cluster that an algorithm can find may be used as for selecting the cluster of accepted signals. Any one of these measures may be used, which may lead to slightly different clusters, each of which can be used to identify acceptable signals.

Digital signal processing circuit 26 may use any one of various different other known clustering techniques to cluster the signals from A/D converters 24 into clusters of similar signals, the largest cluster being used as cluster of accepted samples. One other clustering technique, for example, may comprise computing distance measures between signals from pairs of electrodes, using each electrode as an initial cluster and merging clusters if the smallest distance measure between the signals in these clusters are below a threshold.

It should be appreciated that the algorithm of figure 3 provides an example of a clustering algorithm. Both distance and correlations are examples of measures of similarity. The distance based on a sum of squares of R(i,t)-R(j,t+dt) and the correlation are closely related: increasing distance corresponds to decreasing correlation. Selecting pairs of signals that have correlations below a threshold corresponds to identifying pairs that cause a diameter of the cluster of accepted signals to lie above a threshold (the diameter of a cluster is the maximum distance between any pair of elements in the cluster). Removing the sensor with high correlation to the fewest of the sensors in the cluster corresponds to splitting the cluster to reduce the diameter while maintaining maximum connectivity within the cluster.

Although an algorithm has been described for identifying acceptable signals by determining a cluster with no more than a predetermined diameter and a maximum cardinality (number of sensors), it should be appreciated that alternatively other criteria for selecting the cluster may be used, such as a cluster with highest density (cardinality divided by any increasing function of diameter) or a minimum diameter with at least a predetermined cardinality.

Although an example of an application to storage of selected signals has been described (for which purpose digital signal processing circuit may be provided with a non-volatile memory 29 for example, or in a disk drive memory, a battery back-up memory etc.), it should be appreciated that other applications are possible For example, one or more signals in the cluster of accepted signals may be used to detect a physiological state of the person wearing the piece of clothing and to generate an alarm signal when a predetermined condition on the physiological state is satisfied. As another example digital signal processing circuit 26 may be configured to store data derived from signals from A/D converters 24 only if such a predetermined condition is satisfied. As yet another example the arrangement may be provided with a device for applying treatment to the person wearing the clothing if such a predetermined condition is met (e.g. by applying an electric pulse to the skin of the person).

Although an example has been described wherein capacitive electrodes were used to for capacitive sensors to measure signals from the body, it will be appreciated that other types of sensor are possible. For example instead of the electrodes body temperature sensors, resistance sensors, perspiration sensors or combinations thereof may be used. In each case temporary removal of the sensors from the body may be detected by clustering signals from the body and accepting signals from a cluster of similar signals.

As described the required signal processing operations are executed by digital signal processing circuit 26. Digital signal processing circuit 26 may contain a programmable signal processor for example programmed with a program with instructions that will cause the programmable signal processor to perform the operations described in the preceding. As an alternative dedicated circuits especially designed to perform these operations may be used or a combination of dedicated circuits to perform part of the operations and a programmed circuit to perform remaining parts or a distributed cluster of programmable processors etc.

In an embodiment digital signal processing circuit 26 is worn in the piece of clothing. A wireless link may be provided between the sensors and digital signal processing circuit 26 to transmit signal data. In this case digital signal processing circuit 26 may be located remote from the piece of clothing. The computations may be performed real-time as signal values come in, but alternatively signal values may be stored temporarily in a buffer memory and processed later.

## Claims

1. A body parameter sensing arrangement comprising
clothing (10),
a plurality of sensors (12, 20) for sensing body signals, located at mutually movable relative positions in the clothing (10), and
a processing circuit (26) coupled to the plurality of sensors (12, 20), the processing circuit (26) being configured to identify selected ones of the sensors (12, 20) that carry valid body signals by clustering the sensors (12, 20) according to a measure of similarity between signals from the sensors (12, 20) and to use a cluster of sensors (12, 20) with a maximal count of sensors (12, 20) within a minimal cluster diameter, the cluster diameter being defined as maximum distance between the signals from any pair of sensors in the cluster, a correlation between the signals being indicative of the distance, to select sensors (12, 20) that carry valid body signals.

2. A body parameter sensing arrangement according to claim 1, wherein the processing circuit (26) is configured to compute values of a measure of similarity between signals for pairs of sensors (12, 20), with a respective mutual time offset between the signals for each pair, the respective time offsets being settable to mutually different values.

3. A body parameter sensing arrangement according to claim 1, wherein the processing circuit (26) is configured to select the time offsets dynamically according to a criterion that maximizes the similarity between the signals.

4. A body parameter sensing arrangement according to claim 1, wherein the processing circuit (26) is configured to compute correlations between signals from pairs of the sensors.

5. A body parameter sensing arrangement according to claim 4, wherein the processing circuit (26) is configured to search for pairs of sensors (12, 20) in the cluster for which pair the signals in the pair have correlations below a threshold and to remove each time one of the sensors (12, 20) in the pair from the cluster, which removed sensor (12, 20) has correlations above a threshold with less of the sensors (12, 20) than the other sensor (12, 20) in the pair.

6. A body parameter sensing arrangement according to claim 1, wherein the processing circuit (26) is configured to compute an average of signals for sensors (12, 20) from only the cluster.

7. A body parameter sensing arrangement according to claim 6, wherein the processing circuit (26) is configured to filter each of said individual signals from said cluster and to compute the average of signals for sensors (12, 20) from the filtered signals.

8. A body parameter sensing arrangement according to claim 6, comprising a non-volatile memory (29), the processing circuit (26) being configured to store the average in the non-volatile memory (29).

9. A body parameter sensing arrangement according to claim 1, wherein the sensors comprise capacitive electrodes (20) configured to pick up potential changes on locations of the body.

10. A body parameter sensing arrangement according to claim 1, wherein the processing circuit (26) is configured to perform said clustering repeatedly to form respective clusters for respective time intervals to identify the selected ones of the sensors (12, 20) that carry valid body signals in the respective time intervals.

11. A method of processing body signals from a plurality of sensors for sensing body signals, located at mutually movable relative positions in the clothing the method comprising clustering the sensors according to a measure of similarity between signals from the sensors (12, 20) and to use a cluster of sensors (12, 20) with a maximal count of sensors (12, 20) within a minimal cluster diameter, the cluster diameter being defined as maximum distance between the signals from any pair of sensors in the cluster, a correlation between the signals being indicative of the distance to select sensors (12, 20) that carry valid body signals.

## Patentansprüche

1. Anordnung zur Körperparametererfassung, die Folgendes umfasst:
Kleidung (10),
eine Vielzahl von Sensoren (12, 20) zum Erfassen von Körpersignalen, die an zueinander bewegbaren relativen Stellen in der Kleidung (10) angeordnet sind, und
eine Verarbeitungsschaltung (26), die mit der Vielzahl von Sensoren (12, 20) gekoppelt ist, wobei die Verarbeitungsschaltung (26) konfiguriert ist, um ausgewählte der Sensoren (12, 20), welche gültige Körpersignale führen, zu identifizieren, indem die Sensoren (12, 20) gemäß einem Ähnlichkeitsmaß unter den Signalen von den Sensoren (12, 20) gruppiert werden, und um eine Gruppe von Sensoren (12, 20) mit einer maximalen Anzahl von Sensoren (12, 20) innerhalb eines minimalen Gruppendurchmessers zu verwenden, wobei der Gruppendurchmesser als maximaler Abstand zwischen Signalen von einem beliebigen Sensorenpaar in der Gruppe definiert wird, wobei eine Korrelation zwischen den Signalen den Abstand angibt, um Sensoren (12, 20) auszuwählen, die gültige Körpersignale führen.

2. Anordnung zur Körperparametererfassung nach Anspruch 1, wobei die Verarbeitungsschaltung (26) konfiguriert ist, um Werte eines Ähnlichkeitsmaßes unter Signalen für Paare von Sensoren (12, 20) zu berechnen, und zwar mit einem jeweiligen Zeitversatz zwischen den Signalen für jedes Paar, wobei die jeweiligen Zeitversätze auf voneinander unterschiedliche Werte eingestellt werden können.

3. Anordnung zur Körperparametererfassung nach Anspruch 1, wobei die Verarbeitungsschaltung (26) konfiguriert ist, um die Zeitversätze dynamisch entsprechend einem Kriterium auszuwählen, das die Ähnlichkeit zwischen den Signalen maximiert.

4. Anordnung zur Körperparametererfassung nach Anspruch 1, wobei die Verarbeitungsschaltung (26) konfiguriert ist, um Korrelationen zwischen Signalen von Sensorenpaaren zu berechnen.

5. Anordnung zur Körperparametererfassung nach Anspruch 4, wobei die Verarbeitungsschaltung (26) konfiguriert ist, um nach Sensorpaaren (12, 20) in der Gruppe zu suchen, bei denen die Signale in dem Paar Korrelationen unter einem Schwellenwert haben und um jedes Mal einen der Sensoren (12, 20) in dem Paar aus der Gruppe zu entfernen, wobei der entfernte Sensor (12, 20) Korrelationen über einem Schwellenwert mit weniger der Sensoren (12, 20) hat als der andere Sensor (12, 20) in dem Paar.

6. Anordnung zur Körperparametererfassung nach Anspruch 1, wobei die Verarbeitungsschaltung (26) konfiguriert ist, um einen Mittelwert von Signalen für Sensoren (12, 20) aus nur der Gruppe zu berechnen.

7. Anordnung zur Körperparametererfassung nach Anspruch 6, wobei die Verarbeitungsschaltung (26) konfiguriert ist, um jedes der genannten einzelnen Signale aus der genannten Gruppe zu filtern und um den Mittelwert von Signalen für Sensoren (12, 20) aus den filterten Signalen zu berechnen.

8. Anordnung zur Körperparametererfassung nach Anspruch 6, mit einem nicht-flüchtigen Speicher (29), wobei die Verarbeitungsschaltung (26) konfiguriert ist, um den Mittelwert in dem nicht-flüchtigen Speicher (29) zu speichern.

9. Anordnung zur Körperparametererfassung nach Anspruch 1, wobei die Sensoren kapazitive Elektroden (20) umfassen, die konfiguriert sind, um Potentialänderungen an Körperstellen zu erfassen.

10. Anordnung zur Körperparametererfassung nach Anspruch 1, wobei die Verarbeitungsschaltung (26) konfiguriert ist, um die genannte Gruppenbildung wiederholt durchzuführen, um jeweilige Gruppen für jeweilige Zeitintervalle zu bilden, um die ausgewählten der Sensoren (12, 20) zu identifizieren, welche in den betreffenden Zeitintervallen Körpersignale führen.

11. Verfahren zum Verarbeiten von Körpersignalen von einer Vielzahl von Sensoren zum Erfassen von Körpersignalen, die an zueinander bewegbaren relativen Stellen in der Kleidung angeordnet sind, wobei das Verfahren das Gruppieren der Sensoren gemäß einem Ähnlichkeitsmaß unter den Signalen von den Sensoren (12, 20) umfasst, und um eine Gruppe von Sensoren (12, 20) mit einer maximalen Anzahl von Sensoren (12, 20) innerhalb eines minimalen Gruppendurchmessers zu verwenden, wobei der Gruppendurchmesser als maximaler Abstand zwischen den Signalen von einem beliebigen Sensorenpaar in der Gruppe definiert wird, wobei eine Korrelation zwischen den Signalen den Abstand angibt, um Sensoren (12, 20) auszuwählen, die gültige Körpersignale führen.

## Revendications

1. Dispositif de détection de paramètres corporels, comprenant :
un article d'habillement (10),
une pluralité de capteurs (12, 20) destinés à détecter des signaux corporels, situés à des positions relatives mutuellement mobiles dans l'article d'habillement (10), et
un circuit de traitement (26) couplé à la pluralité de capteurs (12, 20), le circuit de traitement (26) étant configuré de sorte à identifier, parmi les capteurs (12, 20), des capteurs sélectionnés qui transportent des signaux corporels valides, en regroupant les capteurs (12, 20) en fonction d'une mesure de similitude entre les signaux issus des capteurs (12, 20), et de sorte à utiliser un groupe de capteurs (12, 20) regroupant un nombre maximal de capteurs (12, 20) au sein d'un diamètre minimal du groupe, le diamètre du groupe étant défini comme la distance maximale entre les signaux issus de n'importe quelle paire de capteurs dans le groupe, une corrélation entre les signaux indiquant la distance, afin de sélectionner les capteurs (12, 20) transportant des signaux corporels valides.

2. Dispositif de détection de paramètres corporels selon la revendication 1, dans lequel le circuit de traitement (26) est configuré de sorte à calculer les valeurs d'une mesure de similitude entre signaux pour des paires de capteurs (12, 20), avec un décalage temporel respectif mutuel entre les signaux pour chaque paire, les décalages temporels respectifs pouvant être déterminés à des valeurs mutuellement différentes.

3. Dispositif de détection de paramètres corporels selon la revendication 1, dans lequel le circuit de traitement (26) est configuré de sorte à sélectionner les décalages temporels de manière dynamique en fonction d'un critère qui maximise la similarité entre les signaux.

4. Dispositif de détection de paramètres corporels selon la revendication 1, dans lequel le circuit de traitement (26) est configuré de sorte à calculer des corrélations entre signaux issus de paires de capteurs.

5. Dispositif de détection de paramètres corporels selon la revendication 4, dans lequel le circuit de traitement (26) est configuré de sorte à rechercher parmi les paires de capteurs (12, 20) dans le groupe, la paire dont les signaux présentent des corrélations inférieures à une valeur seuil, et de sorte à retirer à chaque fois dans le groupe un des capteurs (12, 20) de la paire, ledit capteur retiré (12, 20) présentant des corrélations supérieures à une valeur seuil avec moins de capteurs (12, 20) que l'autre capteur (12, 20) de la paire.

6. Dispositif de détection de paramètres corporels selon la revendication 1, dans lequel le circuit de traitement (26) est configuré de sorte à calculer une moyenne de signaux uniquement pour les capteurs (12, 20) du groupe.

7. Dispositif de détection de paramètres corporels selon la revendication 6, dans lequel le circuit de traitement (26) est configuré de sorte à filtrer chacun desdits signaux individuels issus dudit groupe, et de sorte à calculer la moyenne des signaux pour les capteurs (12, 20) à partir des signaux filtrés.

8. Dispositif de détection de paramètres corporels selon la revendication 6, comprenant une mémoire non volatile (29), le circuit de traitement (26) étant configuré de sorte à enregistrer la moyenne dans la mémoire non volatile (29).

9. Dispositif de détection de paramètres corporels selon la revendication 1, dans lequel les capteurs comprennent des électrodes capacitives (20) configurées de sorte à capter les changements de potentiel à des emplacements du corps.

10. Dispositif de détection de paramètres corporels selon la revendication 1, dans lequel le circuit de traitement est configuré de sorte à effectuer ledit regroupement de manière répétée pour former des groupes respectifs pour des laps de temps donnés, afin d'identifier, parmi les capteurs, les capteurs sélectionnés (12, 20) qui transportent des signaux corporels valides pendant les laps de temps respectifs.

11. Procédé de traitement de signaux corporels issus d'une pluralité de capteurs destinés à détecter des signaux corporels situés à des positions relatives mutuellement mobiles dans l'article d'habillement, le procédé comprenant les étapes consistant à regrouper les capteurs en fonction d'une mesure de similitude entre les signaux issus des capteurs (12, 20), et utiliser un groupe de capteurs (12, 20) regroupant un nombre maximal de capteurs (12, 20) au sein d'un diamètre minimal du groupe, le diamètre du groupe étant défini comme la distance maximale entre les signaux issus de n'importe quelle paire de capteurs dans le groupe, une corrélation entre les signaux indiquant la distance, afin de sélectionner les capteurs (12, 20) transportant des signaux corporels valides.
